Europäisches Patentamt

European Patent Office

Office européen des brevets

(19)

(11) **EP 0 803 731 B1**

(12) **EUROPÄISCHE PATENTSCHRIFT**

(45) Veröffentlichungstag und Bekanntmachung des Hinweises auf die Patenterteilung:
**03.03.2004 Patentblatt 2004/10**

(51) Int Cl.$^7$: **G01N 33/18**, G01N 31/00

(21) Anmeldenummer: **97105881.3**

(22) Anmeldetag: **10.04.1997**

(54) **Verfahren und Vorrichtung zur kontinuierlichen Bestimmung gasförmiger Oxidationsprodukte**

Method and apparatus for continuous determination of gaseous oxidation products

Méthode et appareil pour la détermination en continu de produits d'oxydation en forme de gaz

(84) Benannte Vertragsstaaten:
**AT BE CH DE DK ES FR GB GR IE IT LI LU MC NL PT SE**

(30) Priorität: **26.04.1996 DE 19616760**

(43) Veröffentlichungstag der Anmeldung:
**29.10.1997 Patentblatt 1997/44**

(73) Patentinhaber: **Fraunhofer-Gesellschaft zur Förderung der angewandten Forschung e.V. 80686 München (DE)**

(72) Erfinder:
• **Hambitzer, Günther, Dr.-Rer.-Nat. 76327 Pfinztal (DE)**
• **Liehmann, Wolfgang, Dipl.-Phys. 76356 Weingarten (DE)**
• **Beurer, Bernhard, Dipl.-Ing. 76137 Karlsruhe (DE)**

(56) Entgegenhaltungen:
**US-A- 3 840 341      US-A- 4 141 829
US-A- 4 293 522      US-A- 5 132 094
US-A- 5 292 666      US-A- 5 340 542
US-A- 5 413 763**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren nach dem Oberbegriff des Anspruchs 1 sowie eine Vorrichtung nach dem Oberbegriff des Anspruchs 12.

Der TOC-Wert (Total Organic Carbon = gesamter organisch gebundener Kohlenstoff) ist eine wichtige Kerngröße für die Belastung eines Abwassers mit organischen Stoffen. Die Messung dieses Wertes liefert darüber hinaus Leitwerte für die Bestimmung des CSB-Wertes (chemischer Sauerstoffbedarf) und des BSB-Wertes (biologischer Sauerstoffbedarf). Die Anwendungsbereiche zur Bestimmung des TOC-Wertes liegen daher im Bereich des Umweltschutzes, insbesondere der Abwasserüberwachung, sowie darüber hinaus der Prozeßregelung und Überwachung im industriellen Bereich.

Bei bekannten Vorrichtungen erfolgt die Bestimmung des TOC-Wertes über eine oxidative Umsetzung des Kohlenstoffs orangischer Verbindungen zu messbarem Kohlendioxid. Dabei müssen die zu untersuchenden Probelösungen vor der eigentlichen Messung bei der Probennahme filtiert und Mehrphasengemische müssen aufgearbeitet werden, um Ablagerungen, die zu einer Erniedrigung des Nachweissignals sowie einer Reduzierung der Ansprechzeit führen, in der Vorrichtung zu verhindern.

Neben den anorganischen Verbindungen sind in den meisten zu untersuchenden Proben auch kohlenstoffhaltige anorganische Verbindungen, überwiegend in Form von Carbonaten und Hydrogencarbonaten, enthalten. Der Wert des gesamten anorganisch gebundenen Kohlenstoffs (= Total Inorganic Carbon) wird auch als TIC-Wert bezeichnet. Häufig werden die kohlenstoffhaltigen anorganischen Verbindungen vor der Bestimmung des TOC-Wertes unter Zugabe von Säure (pH<2) zur Probelösung unter Verwendung eines kohlendioxidfreien Trägergases entfernt. Dabei werden aber auch leichtflüchtige Kohlenwasserstoffe mit ausgestrippt. Sollen diese leichtflüchtigen Kohlenwasserstoffe jedoch bei der Bestimmung berücksichtigt werden, müssen sie nach vorheriger Abtrennung der anorganischen Verbindungen der Oxidationseinheit zugeführt werden.

Die eigentliche oxidative Umsetzung der organischen Verbindungen erfolgt bei den meisten sich derzeit auf dem Markt befindlichen Prozeß-TOC-Geräten durch UV-Bestrahlung in Kombination mit einem Oxidationsmittel oder aber durch thermisch katalytische Oxidation.

Geräte mit UV-Aufschlusseinheiten sind nicht in der Lage, den organisch gebundenen Kohlenstoff schwer oxidierbarer organischer Verbindungen vollständig zu Kohlendioxid umzusetzen. Der organisch gebundene Kohlenstoff dieser Verbindungen kann deshalb auch nicht detektiert werden und führt zu Minderbefunden und somit zu unsicheren Analyseergebnissen. Bei Probenlösungen mit einem hohen Chloridanteil führt die UV-Bestrahlung überdies zur Bildung von Chlor, welches wiederum UV-Strahlung absorbiert und die Nachweisempfindlichkeit des Gerätes herabsetzt.

Aus diesem Grunde werden zunehmend Geräte eingesetzt, bei denen die Umsetzung zu Kohlendioxid durch eine katalytisch gestützte thermische Oxidation erfolgt. Bei geeigneter Wahl der Temperatur des Katalysators sowie einer ausreichenden Verweilzeit der Probe im Ofen ist die Oxidation unabhängig von der Probe und liefert eine nahezu hundertprozentige Nachweisempfindlichkeit für alle Kohlenwasserstoffverbindungen. Nachteilig ist jedoch, dass die zur Oxidation notwendigen hohen Temperaturen im Bereich von 850 °C zu Ablagerungen in der Ofeneinheit und damit zu einem erhöhten Verschleiß derselben führen. Des weiteren werden als Katalysatoren solche aus Platin (Pt) oder Palladium (Pd) verwendet, die teuer sind und regelmäßig ausgetauscht werden müssen. Da die Oxidationsprodukte mit Hilfe eines Trägergasstroms über entsprechende Reinigungsstufen einer Detektionseinheit zur Detektion des Kohlendioxids zugeführt werden müssen, treten insbesondere bei Probenlösungen mit einem hohen Anteil an brennbaren Flüssigkeiten Probleme mit der Detektion auf, weil diese in der druckabhängigen Oxidationseinheit zu extremen Druckänderungen führen können. Als Detektoren werden im allgemeinen nichtdispersive IR-Detektoren'verwendet, die jedoch auf die Detektion von Kohlendioxid allein beschränkt sind.

Die US 4,141,829 zeigt ein Verfahren zur Bestimmung gasförmiger Oxidationsprodukte aus Wasser, insbesondere zur Bestimmung gasförmiger Oxidationsprodukte aus Wasser, insbesondere zur Bestimmung des TOC-Wertes, wobei organisch gebundener Kohlenstoff oxidativ umgesetzt wird und die gasförmigen Oxidationsprodukte bestimmt werden. Hierzu wird die Probelösung kontinuierlich in einen Reaktor gepumpt und dort auf eine vorgegebene Temperatur erhitzt und die organischen Verbindung in der Probelösung werden unter definiert erhöhter Temperatur und definiert erhöhtem Druck in chemischer Oxidation oxidativ umgesetzt.

Die genannte Druckschrift sowie die US 3,840,241 zeigen Reaktorsysteme mit beheizbaren Reaktoren die weiterhin eine Pumpe enthalten.

Der Erfindung liegt die Aufgabe zugrunde, ein Verfahren und eine Vorrichtung der eingangs genannten Art zu schaffen, mittels denen in bevorzugter Weise zumindest der TOC-Wert in Prozeß-, Trink- und/oder Abwasser kontinuierlich und zuverlässig bestimmt werden kann.

Erfindungsgemäß wird die genannte Aufgabe mit einem Verfahren der gattungsgemäßen Art gelöst, welches weiterhin die kennzeichnenden Merkmale des Anspruchs 1 aufweist.

Darüber hinaus wird die genannte Aufgabe mit einer gattungsgemäßen Vorrichtung gelöst, die die kennzeichnenden Merkmale des Anspruchs 12 aufweist.

Es hat sich überraschend herausgestellt, dass mittels des erfindungsgemäßen Verfahrens sowie der erfindungsgemäßen Vorrichtung eine kontinuierliche Umsetzung organischer Verbindungen, auch schwer oxidierbarer Verbindungen, möglich ist. Die zur Umsetzung notwendige Temperatur wird dabei durch das Beheizen des Mikroreaktors erreicht. Der zur Umsetzung notwendige, erhöhte Druck wird dabei bevorzugt durch den Verdampfungsdruck der Probelösung in Form einer Kapillare geringen Durchmessers ausgebildeten Mikroreaktors aufgebaut. Auf diese Weise kann der Druckaufbau im Mikroreaktor bzw. der Kapillare kontinuierlich durch das schnelle Verdampfen der erhitzten Probelösung und dem daraus resultierenden Dampfdruck erfolgen, wobei überkritische Bedingungen entstehen. Der Mikroreaktor kann hierzu direkt oder auch indirekt beheizt werden. Hierzu ist die Kapillare entweder selbst als Heizwiderstand ausgebildet oder aber steht mit Heizkörpermaterial einer Widerstandsheizung in wärmeleitender Verbindung.

Zur Detektion der gebildeten Oxidationsprodukte wird die zu untersuchende Probe direkt und kontinuierlich unter überkritischen Bedingungen also oxidativ umgesetzt. Es ist kein Trägergasstrom zum Transport der Probe durch die Oxidationseinheit und zum Detektor erforderlich, da die flüchtigen Oxidationsprodukte ohne vorherigen Phasentransfer direkt aus der Probe bestimmt werden. Als Oxidationstemperatur reichen bereits Temperaturen von ca. 400 °C aus, während die Temperaturen bei der katalytischen Verbrennung höher liegen. Höhere Temperaturen sind denkbar, wenn die Reaktionskapillaren über eine ausreichende Temperaturbeständigkeit und Korrisionsfestigkeit verfügen. Es ist bereits ein Druck von ca. 250 bar zur Oxidation der organischen Verbindungen ausreichend.

In Weiterbildung ist vorgesehen, dass der Probelösung Sauerstoff zugeführt wird. Die Zuführung des Sauerstoffs kann dabei vor bzw. während der oxidativen Umsetzung erfolgen. Hierdurch ist eine vollständige Umsetzung aller organischen Verbindungen in der Probelösung gewährleistet. Der Sauerstoff wird dabei bevorzugt elektrochemisch erzeugt, es ist aber auch eine Zuführung über externe Gaszuführungen möglich. Die elektrochemische Sauerstofferzeugung kann dabei direkt im Reaktorinneren durch Elektroden mit entsprechender Geometrie oder aber außerhalb erfolgen. Aufgrund der Reaktionsbedingungen (Druck, Temperatur) im Inneren des Mikroreaktors werden die organischen Verbindungen in der Probelösung und der zugeführte Sauerstoff vollständig gelöst. Da nun die oxidative Umsetzung in einem einphasigen System stattfindet, sind die Reaktionsbedingungen hier besser kontrollierbar als bei bekannten Verbrennungsreaktionen. Da der Sauerstoff vorzugsweise im Überschuß zugeführt wird, werden die organischen Verbindungen zu Kohlendioxid und Wasser oxidiert. Enthaltene Heteroatome in den Kohlenwasserstoffverbindungen (Schwefel, Halogene und Stickstoff) werden in die entsprechenden Mineralsäuren umgewandelt.

Anschließend wird die in die gasförmige Phase übergeführte Probelösung vorzugsweise kondensiert. Die kondensierte Probe wird dann weiterbildend mit konstanter Fließgeschwindigkeit durch eine Durchflußzelle geleitet. Vorher wird dieser bevorzugt Säure zudosiert, um sicherzustellen, daß in der kondensierten Probe enthaltenes Hydrogencarbonat und Carbonat in freies Kohlendioxid (frei Kohlensäure) umgewandelt wird. Um eine vollständige Umsetzung des Hydrogencarbonats und Carbonats sicherzustellen, sollte der pH-Wert nach der Säurezugabe bei pH = 2 liegen. Da die Konzentration der freien Kohlensäure sowie die Konzentration der Carbonate und Hydrogencarbonate direkt vom pH-Wert abhängig sind und mit dem erfindungsgemäßen Verfahren sowie der erfindungsgemäßen Vorrichtung nur gasförmige Stoffe erfaßt werden können, muß für die Bestimmung des Kohlendioxids der pH-Wert bekannt sein oder konstant gehalten werden. Bei einem nicht konstanten pH-Wert kann die Bestimmung des Kohlendioxids alternativ durch die Messung der freien Kohlensäure (Kohlendioxid) sowie einer Messung des pH-Wertes erfolgen.

Um nun die gasförmigen Oxidationsprodukte einer Analyse- bzw. Detektionseinheit zuzuführen, wird die kondensierte Probe an einer flüssigkeitsundurchlässigen, aber gasdurchlässigen Membran vorbeigeführt, die gasförmigen Oxidationsprodukte werden in ihrer Gasphase aus der Probe durch die Membran abgesaugt und durch eine Meßzelle geleitet. Bevorzugt wird die Probe dabei im Bereich der Membran auf einer konstanten Temperatur und einem vorgegebenen Druck gehalten. Durch diese definierten Temperatur- und Druckbedingungen in der Durchflußzelle ist die Menge an gelöstem Kohlendioxid im Kondensat, also der kondensierten Probe, der Menge an gasförmigem Kohlendioxid in der Detektionseinheit proportional. Ein Anteil der zu untersuchenden, gasförmigen Oxidationsprodukte gelangt dabei aus einem Bereich geringen Unterdrucks über einen Druckwandler mit einer Blende zu der Detektionseinheit. Der Druckwandler sollte dabei auf der gleichen konstanten Temperatur wie die Durchflußkammer gehalten werden. Durch den Unterdruck wird das Diffusionsverhalten der gasförmigen Oxidationsprodukte durch die Membran hindurch unterstützt und verstärkt.

In Weiterbildung ist vorgesehen, daß die gasförmigen Oxidationsprodukte mittels eines Massenspektrometers gemessen werden. Durch das Massenspektrometer ergibt sich die Möglichkeit, auch andere gasförmige Oxidationsprodukte als lediglich Kohlendioxid, beispielsweise aus Halogen-, Schwefel- oder Stickstoffverbindungen stammende Oxidationsprodukte, zu erfassen. Damit kann die Analyseneinheit nach entsprechender Anpassung beispielsweise auch zur Bestimmung des Gesamtstickstoffgehaltes nach Kjeldahl verwendet werden.

Aus Kostengründen können die gasförmigen Oxidationsprodukte beispielsweise auch mittels eines nichtdispersiven Infrarotdetektors oder eines Flammenionisationsdetektors bestimmt werden. Diese Gasanalysatoren sind jedoch nur für den Nachweis von gasförmigem Kohlendioxid geeignet.

Die erfindungsgemäße Vorrichtung sieht zur Sauerstoffeinspeisung bzw. -erzeugung bevorzugt einen dem Mikroreaktor vorgeordneten elektrochemischen Reaktor vor. Alternativ können im Inneren des Mikroreaktors angeordnete Elektroden zur elektrochemischen Sauerstofferzeugung vorgesehen sein. Die Sauerstofferzeugung erfolgt dann also direkt im Mikroreaktorinneren durch Elektroden mit entsprechender Geometrie. Neben der elektrochemischen Erzeugung des Sauerstoffs ist aber auch eine direkte Zudosierung von reinem Sauerstoff möglich. Hierzu sieht die Vorrichtung dann eine Gaszuführung zur Einspeisung von Sauerstoff vor.

Während der für die oxidative Umsetzung notwendige überkritische Druck bevorzugt durch den Verdampfungsdruck der Probelösung im Inneren der Kapillare aufgebaut wird, ist bei einer anderen Ausführungsform ein dem Mikroreaktor vorgeschaltetes Druckhalteventil, wie ein Regulierventil, vorgesehen. Der Druckaufbau erfolgt dabei durch eine Verringerung des Strömungsquerschnitts am offenen Ende des Mikroreaktors. Bei Verwendung eines solchen Druckhalteventils direkt hinter dem Mikroreaktor für den Druckaufbau ist dann die Geometrie der Kapillare bzw. des Mikroraktors veränderbar. Insbesondere können durch die Vergrößerung des Reaktorvolumens längere Reaktionszeiten realisiert werden.

In bevorzugter Ausgestaltung ist vorgesehen, daß die Innenrohrwände des Mikroreaktors temperatur- und korrosionsbeständig beschichtet sind. Als Beschichtungsmaterial kommen dabei Metallegierungen in Frage, die eben über eine solche ausreichende Temperaturfestigkeit sowie Korrosionsbeständigkeit verfügen. Vorzugsweise kommen hierfür Nickel und Nickellegierungen sowie auch Tantal in Betracht. Diese Materialien weisen neben der chemischen Beständigkeit auch katalytische Eigenschaften auf. Die Beschichtung der Innenrohrwände kann aber auch mit anderen Katalysatormaterialien erfolgen, die ähnliche Eigenschaften wie die vorgenannten aufweisen. Für die Außenrohrwände können als temperaturbeständige Werkstoffe spezielle temperaturbeständige Stähle (z.B. Cr-Ni-No-Stahl) oder keramische Materialien verwendet werden, die über eine ausreichende mechanische Festigkeit verfügen. Bei Verwendung von Werkstoffkombinationen bestehen die äußere Rohrwandung und die Kapillare dabei aus dem gleichen Material.

In Weiterbildung ist dem Mikroreaktor eine Kondensatoreinheit nachgeordnet. Diese Einheit kann in Form eines ausreichend dimensionierten Kühlers zur Kondensation der nun gasförmigen Probe ausgebildet sein.

Um nun die Probe einer Detektionseinheit zuführen zu können, ist bevorzugt eine Pumpeneinrichtung zum Pumpen der kondensierten Probe durch eine Durchflußkammer oder -zelle vorgesehen. Um einen kontinuierlichen Durchfluß zu erreichen, kann weiterhin vorgesehen sein, daß der Durchflußzelle ein Druckhalteventil nachgeordnet ist. Hierdurch wird zwischen der Pumpe und dem offenen Ende der Durchflußzelle ein konstanter Druck aufgebaut, um beispielsweise gasförmiges Kohlendioxid in Lösung zu halten.

Bevorzugt ist zwischen der Kondensationseinheit und der Durchflußzelle eine Stelle zur Säureeinspeisung vorgesehen. Aufgrund der dadurch möglichen Säurezudosierung wird sichergestellt, daß in der Probe enthaltenes Hydrogencarbonat und Carbonat in freies Kohlendioxid umgewandelt wird.

Um die in der kondensierten Probe enthaltenen gasförmigen Oxidationsprodukte einer Messung zuzuführen, ist die Durchflußzelle bevorzugt durch eine flüssigkeitsundurchlässige, aber gasdurchlässige Membran von einer Meßzelle getrennt. Um das Hindurchtreten der zu untersuchenden gasförmigen Oxidationsprodukte aus der kondensierten Probe durch die Membran zu unterstützen, sehen Weiterbildungen vor, daß an der Durchflußzelle eine geregelte Heizeinrichtung angeordnet ist und/oder auf der der Durchflußzelle abgewandten Seite der Membran zumindest eine Pumpe vorgesehen ist, die einen mäßigen Unterdruck zum Absaugen zumindest des gasförmigen Kohlendioxids erzeugt. Durch die definierten Temperatur- und Druckbedingungen in der Durchflußzelle ist die Menge an gelöstem Kohlendioxid in der kondensierten Probe proportional zur Menge an gasförmigem Kohlendioxid in der nachfolgenden Detektionseinheit.

In äußerst bevorzugter Ausgestaltung ist dabei weiterhin ein Druckwandler mit einstellbarer Blende vorgesehen. Über diesen gelangt dann ein Teilstrom der gasförmigen Oxidationsprodukte, insbesondere des gasförmigen Kohlendioxids, zur Detektionseinheit. Die Meßzelle weist hierzu vorzugsweise eine Elektronenstoßionenquelle eines Quadrupolmassenspektrometers auf. Dieses Massenspektrometer bietet die Möglichkeit, auch andere gasförmige Oxidationsprodukte zu bestimmen, die aus Halogen-, Schwefel- oder Stickstoffverbindungen stammen. So ist beispielsweise nach entsprechender Anpassung auch eine Bestimmung des Gesamtstickstoffgehalts nach Kjeldahl möglich. Durch die Verwendung eines Bypasses an der Oxidationseinheit bzw. durch schnelles Abkühlen der Oxidationseinheit können neben den gasförmigen Oxidationsprodukten auch gasförmige Inhaltsstoffe (beispielsweise CKWs, Phenole und andere) in der Probelösung bestimmt werden.

Als Einheit zur Messung der gasförmigen Oxidiationsprodukte kann im übrigen die aus der DE-OS 41 33 300 bekannte Einrichtung der Anmelderin verwendet werden, so daß auf diese Einrichtung nicht explizit eingegangen werden muß.

Weitere Vorteile und Merkmale der Erfindung ergeben sich aus den Ansprüchen und aus der nachfolgenden Beschreibung, in der Ausführungsbeispiele unter Bezugnahme auf die Zeichnung im einzelnen erläutert sind. Dabei zeigt:

Fig. 1 eine schematische Darstellung der wesentlichen Elemente einer ersten Ausführungsform eines Probenaufbereitungssystems der erfindungsgemäßen Vorrichtung;

Fig. 2 eine zweite Ausführungsform der erfindungsgemäßen Vorrichtung in detaillierter schematischer Darstellung; und

Fig. 3 eine dritte Ausführungsform der erfindungsgemäßen Vorrichtung in detaillierter schematischer Darstellung.

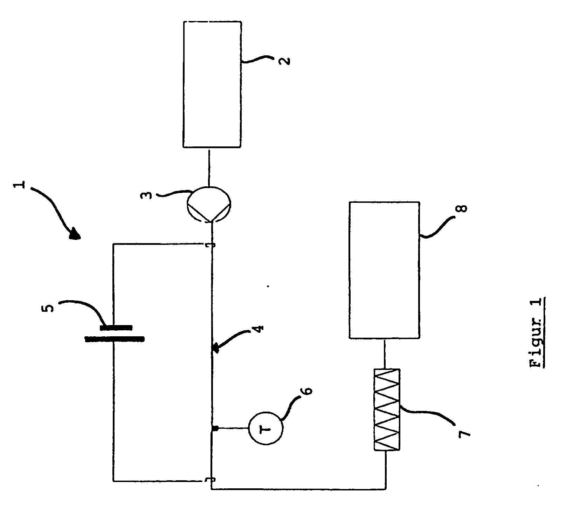

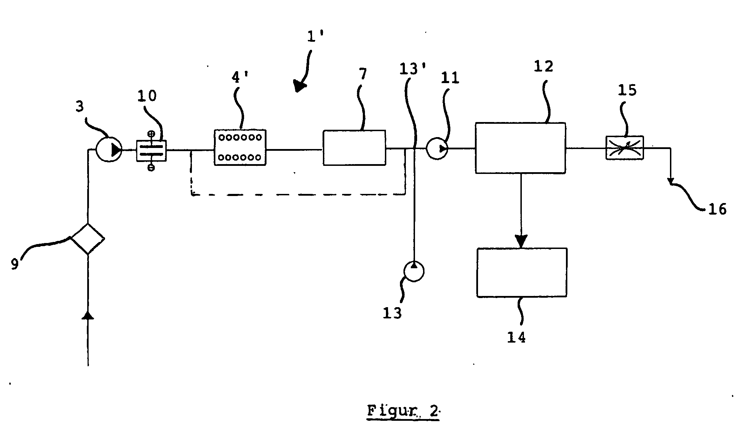

Die in Fig. 1 dargestellte erfindungsgemäße Vorrichtung 1 weist zunächst eine Pumpe 3 auf, mit der eine sauerstoffangereicherte wässrige Probelösung (hierzu Fig. 2) des zu untersuchenden Abwassers einem endseitig geöffneten Mikroreaktor 4, wie einer Reaktionskapillare, zugeführt wird. Die Reaktionskapillare 4 ist aus elektrischem Widerstandsmaterial ausgebildet oder mit solchem Material auf einem dielektrischen, rohrförmigen Träger beschichtet und dient so auch als Heizwiderstand. Sie ist mit einer Gleichspannungsquelle 5 verbunden, mittels der der Mikroreaktor 4 direkt beheizt wird. Zur Überprüfung und gegebenenfalls Regelung der Temperatur ist dabei ein Thermometer 6 vorgesehen.

Der Auslaß der Mikroeinheit 4 ist mit einem Kondensationsgefäß 7 verbunden, in dem die oxidativ umgesetzte Probelösung 2 kondensiert wird. Nach der Kondensation im Kondensationsgefäß 7 liegt die einer Detektionseinheit (nicht dargestellt) zuzuführende kondensierte Probe 8 vor.

Bei der in Fig. 2 dargestellten erfindungsgemäßen Vorrichtung 1' ist der Pumpe 3 - gleiche Bauteile werden mit gleichen Bezugszeichen versehen - eine Filtriereinrichtung 9 vorgeordnet. Zwischen der Pumpe 3 und einer im dargestellten Ausführungsbeispiel indirekt beheizten Oxidationseinheit 4' ist ein elektrochemischer Reaktor 10 zur Sauerstofferzeugung angeordnet. An den Mikroreaktor 4' schließt sich die Kondensationseinheit 7 an. Dieser ist eine Pumpe 11 zur Förderung des Kondensats in und durch eine Durchflußzelle 12 nachgeordnet. Zwischen der Kondensationseinheit 7 und der Pumpe 11 ist eine Einspeisungsstelle 13' zum Einspeisen von Phosphorsäure 13 vorgesehen. Die Durchflußzelle 12 ist schließlich mit einer Analyseneinheit 14, wie einer Meßzelle und einer Detektionseinheit, verbunden. Bei der Analyseneinheit 14 kann es sich dabei um die aus der DE-OS 41 33 300 bekannte handeln. Der Durchflußzelle 12 ist ein Druckhalteventil 15 nachgeordnet, bei dem es sich um ein Regulierventil handelt. Mittels dieses Regulierventils 15 wird ein Druck von p > 5 bar durch Verringerung des Strömungsquerschnitts am Ende der Durchflußzelle 12 aufgebaut. Durch diesen zwischen der Pumpe 11 und der Durchflußzelle 12 konstant gehaltenen Druck wird dann das gasförmige Kohlendioxid in Lösung gehalten. An das Druckhalteventil 15 schließt sich schließlich ein Ablauf 16 an, über den der die Durchflußzelle 12 frei durchfließende Anteil der zu untersuchenden Probe beispielsweise in das Leitungssystem zurückgeführt werden kann.

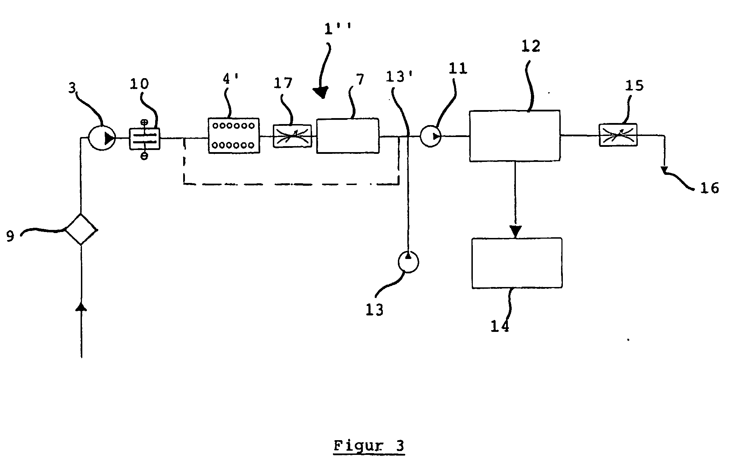

Bei der in Figur 3 dargestellten dritten Ausführungsform einer erfindungsgemäßen Vorrichtung 1" ist zwischen dem Mikroreaktor bzw. der Oxidationseinheit 4' und der Kondensationseinheit 7 ein weiteres Regulierventil 17 als Druckhalteventil angeordnet. Durch das direkt hinter dem Mikroreaktor 4' angeordnete Regulierventil 17 wird der Druck auf p > 223 bar durch Verringerung des Strömungsquerschnitts am offenen Ende des Mikroreaktors 4' aufgebaut. Durch die Verwendung eines solchen Druckhalteventils 17 kann die Geometrie des Mikroreaktors 4' verändert werden, wobei insbesondere durch Vergrößerung des Reaktorvolumens längere Reaktionszeiten realisiert werden können, da der Druckaufbau nicht durch den Dampfdruck der wässrigen Probe im Mikroreaktor 4' erfolgt.

Die Bestimmung des TOC-Wertes erfolgt mittels überkritischer Naßoxidation. Hierzu wird zunächst eine zu untersuchende wässrige Probelösung 2 nach erfolgter Filtration und Sauerstoffanreicherung mittels der Hochdruckpumpe 3 einem indirekt oder direkt beheizten Mikroreaktor 4 bzw. 4' mit einer konstanten Flußrate kontinuierlich zugeführt wird. Bei dem Mikroaktor 4, 4' handelt es sich bevorzugt um eine beheizte Kapillare mit einem geringen Kapillarinnendurchmesser. Innerhalb dieser Kapillare 4',4 wird die Probelösung auf eine vorgegebene Temperatur aufgeheizt, so daß die zur überkritischen Naßoxidation notwendigen physikalischen Parameter (T>373°C und p>225 bar) erreicht werden; die zu untersuchende Probe wird so bei ca. 400 °C und 240 bar unter Zusatz von Sauerstoff oxidativ umgesetzt wird. Der notwendige Druck wird dabei durch den Verdampfungsdruck der Probelösung im Inneren der Kapillare aufgebaut oder aber mittels des Druckhalteventils.

Es ist so eine kontinuierliche Umsetzung der organischen Verbindungen in der Probelösung möglich. Um die vollständige Umsetzung zu gewährleisten, wird der Probelösung mittels des elektrochemischen Reaktors 10 erzeugter Sauerstoff im Überschuß zugeführt. Dies kann dabei nicht nur wie dargestellt vor sondern auch während der oxidativen Umsetzung erfolgen. Aufgrund der Reaktionsbedingungen im Inneren des Mikroreaktors 4, 4' werden die organischen Verbindungen in der Probelösung sowie der Sauerstoff vollständig gelöst. Es wird ein überkritischer Zustand der wässrigen Lösung im Mikroreaktor erzeugt. Die Löslichkeit der zu untersuchenden organischen Verbindungen ist dabei insbesondere aufgrund der homogenen Reaktionsbedingungen im Mikroreaktor wesentlich erhöht. Weiter ergibt sich in diesem Zustand eine drastische Erhöhung der katalytischen Aktivität, die die Reaktionskinetik der Oxidationsreaktion verbessert. Sauerstoff wird als Oxidationshilfsmittel benötigt, um unter den gewählten Bedingungen organische Verbindungen unter Verwendung von Sauerstoff zu Kohlendioxid zu oxidieren. Der Sauerstoff muß im Überschuß vorhanden sein, um eine vollständige Oxidation zu Kohlendioxid zu gewährleisten. Bei einem Sauerstoffmangel können neben Kohlendioxid auch andere nicht erwünschte kohlenstoffhaltige Oxidationsprodukte auftreten.

Die Sauerstofferzeugung erfolgt vorzugsweise elektrochemisch in einer Durchflußzelle vor der Reaktionskapillare. Hierbei wird durch anodische Oxidation des Wassers Sauerstoff erzeugt. Bei der angesprochenen Alternative "während der überkritischen Naßoxidation" müssen die Elektroden in den Reaktorraum integriert werden. Es wird ein Druckhalteventil zur Verbesserung des Druckaufbaus verwendet. Durch die Verwendung eines Druckhalteventils können die Geometrie des Reaktionsraums und der Elektroden frei gewählt werden.

Da die oxidative Umsetzung nun in einem einphasigen System stattfindet, sind die Reaktionsbedingungen hier besser kontrollierbar als bei bekannten Verbrennungsreaktionen. Durch die Zudosierung von Sauerstoff im Überschuß werden die organischen Verbindungen zu Kohlendioxid und Wasser oxidiert. Enthaltene Heteroatome in den Kohlenwasserstoffverbindungen (Schwefel, Halogene und Stickstoff) werden dabei in die entsprechenden Mineralsäuren umgewandelt.

Nach der nun erfolgten oxidativen Umsetzung mittels überkritischer Naßoxidation wird nun die gasförmige Probe in einem ausreichend dimensionierten Kühler der Kondensationseinheit 7 kondensiert. Mittels der Pumpe 11 wird das nun erhaltene Kondensat bzw. die kondensierte Probe 8 der Durchflußzelle 12 zugeführt. Hierbei wird zwischen der Pumpe 11 und dem offenen Ende der Durchflußzelle 12 ein konstanter Druck aufgebaut, um beispielsweise das gasförmige Kohlendioxid in Lösung zu halten. Der Druckaufbau erfolgt dabei über das Druckhalteventil 15.

Zwischen der Kondensationseinheit 7 und der Pumpe 11 erfolgt bei 13' eine Säurezudosierung, beispielsweise die Zudosierung von Phosphorsäure, um in der Probe enthaltenes Hydrogencarbonat und Carbonat in freie Kohlensäure (Kohlendioxid) umzuwandeln. Um eine vollständige Umsetzung des Hydrogencarbonates und der Carbonate sicherzustellen, sollte der pH-Wert nach der Säurezugabe bei pH = 2 liegen. Da die Konzentration der freien Kohlensäure sowie die Konzentration der Carbonate und Hydrogencarbonate direkt vom pH-Wert abhängig sind und mit der verwendeten Meßeinrichtung nur gasförmige Stoffe erfaßt werden können, muß für die Bestimmung des Kohlendioxids der pH-Wert bekannt sein oder konstant gehalten werden. Bei einem nicht konstanten pH-Wert kann die Bestimmung des Kohlendioxids durch die Messung der freien Kohlensäure (Kohlendioxid) sowie eine Messung des pH-Wertes erfolgen.

Die Durchflußzelle 12 dient nun als Probenaufgabesystem für die Analyseeinheit 14. Hierzu ist in der Durchflußzelle 12 eine flüssigkeitsundurchlässige, aber gasdurchlässige Membran angeordnet, durch die zumindest das gasförmige Kohlendioxid mit einer nicht dargestellten Pumpe, die einen konstanten Unterdruck erzeugt, in eine Meßzelle 14 abgesaugt wird. Ein Teilstrom des gasförmigen Kohlendioxids gelangt über einen ebenfalls nicht dargestellten Druckwandler mit einstellbarer Blende zur Detektionseinheit 14. Durch definierte Temperatur- und Druckbedingungen in der Durchflußzelle 12 ist die Menge an gelöstem Kohlendioxid in der kondensierten Probe proportional zur Menge an gasförmigem Kohlendioxid in der Detektionseinheit 14. Zur Detektion wird dabei vorzugsweise ein Massenspektrometer verwendet. Die Meßzelle weist hierzu eine Elektronenstoßionenquelle eines Quadrupolmassenspektrometers auf. Mittels des Massenspektrometers wird nun der Gehalt an Gesamtkohlenstoff, der TC-Wert, bestimmt. Der TOC-Wert an organisch gebundenem Kohlenstoff wird dann durch Differenzbildung zwischen dem TC-Wert an Gesamtkohlenstoff und dem TIC-Wert an anorganisch gebundenem Kohlenstoff ermittelt:

$$TOC = TC - TIC.$$

Der TIC-Wert an Carbonaten, Hydrogencarbonaten und freier Kohlensäure wird vor der Oxidation aus der Probelösung durch Bestimmung des pH-Wertes bestimmt.

Bei einem konkreten Beispiel des erfindungsgemäßen Verfahrens wurde eine schweroxidierbare Substanz, nämlich N-Cetyl-trimethylammoniumbromid, mit dem in Fig. 1 dargestellten Aufbau aufbereitet.

Das schweroxidierbare N-Cetyl-trimethylammoniumbromid, kann bislang nur von Geräten zur Bestimmung des TOC-Wertes mit Verbrennungseinheiten bei hohen Ofentemperaturen über 850 °C vollständig umgesetzt werden. Hierbei ergeben sich aber die bereits vorhergehend aufgeführten Nachteile.

Für die Umsetzung mit der erfindungsgemäßen Oxidationseinheit wurde eine wässrige Standardlösung der Modellsubstanz im ppm-Bereich verwendet. Mit einer handelsüblichen HPLC-Pumpe 3 wurde die Standardlösung 2 mit einer konstanten Flußrate in die als Reaktionskapillare ausgebildete Oxidationseinheit 4 gepumpt. Die gleichzeitig als Heizwiderstand dienende Reaktionskapillare 4, also eine direkt beheizte Oxidationseinheit, wurde dabei auf eine definierte Temperatur aufgeheizt. Durch das Aufheizen der Reaktionskapillare 4 wurde der zur oxidativen Umsetzung benötigte Druckanstieg durch den Verdampfungsdruck der Standardlösung 2 erreicht. Nachfolgend wurde dann die umgesetzte, gasförmige Lösung durch einen entsprechend dimensionierten Kühler 7 kondensiert.

Die Vollständigkeit der oxidativen Umsetzung wurde anschließend durch eine TOC-Analyse (gemäß Fig. 2 oder 3) überprüft. Die Messungen haben dabei gezeigt, daß die Modellsubstanz ohne Sauerstoffzusatz nahezu vollständig umgesetzt wurde.

## Patentansprüche

1.  Verfahren zur kontinuierlichen Bestimmung gasförmiger Oxidationsprodukte aus Prozeß-, Trink- und/oder Abwasser, insbesondere zur Bestimmung des TOC-Wertes, wobei zumindest in einer zu untersuchenden Probelösung enthaltener, organisch gebundener Kohlenstoff oxidativ umgesetzt wird und die gasförmigen Oxidationsprodukte einer Meßeinheit zugeführt werden, in der zumindest die Detektion des organisch gebundenen Kohlenstoffs erfolgt, die Probelösung kontinuierlich auf eine vorgegebene Temperatur erhitzt wird die organischen Verbindungen in der Probelösung unter definiert erhöhter Temperatur und definiert erhöhtem Druck kontinuierlich oxidativ umgesetzt werden, **dadurch gekennzeichnet, dass** die Probelösung kontinuierlich in einen Mikroreaktor gepumpt und dort erhitzt wird, dass der erhöhte Druck durch den Verdampfungsdruck der Probelösung derart aufgebaut wird, dass der Kohlenstoff der zu untersuchenden Probe direkt und kontinuierlich unter überkritischen Bedingungen oxidativ umgesetzt wird.

2.  Verfahren nach Anspruch 1, **dadurch gekennzeichnet, dass** der erhöhte Druck mit Hilfe eines Druckhalteventils aufgebaut wird.

3.  Verfahren nach Anspruch 1 oder 2, **dadurch gekennzeichnet, dass** der Probelösung Sauerstoff zugeführt wird.

4.  Verfahren nach Anspruch 3, **dadurch gekennzeichnet, dass** der Sauerstoff elektrochemisch erzeugt wird.

5.  Verfahren nach einem der Ansprüche 1 bis 4, **dadurch gekennzeichnet, dass** die im Mikroreaktor erzeugte, gasförmige Probe kondensiert wird.

6.  Verfahren nach Anspruch 5, **dadurch gekennzeichnet, dass** der kondensierten Probe Säure zudosiert wird.

7.  Verfahren nach einem der Ansprüche 5 oder 6, **dadurch gekennzeichnet, dass** die kondensierte Probe mit konstanter Fließgeschwindigkeit und unter konstantem Druck durch eine Durchflusszelle geleitet wird.

8.  Verfahren nach Anspruch 7, **dadurch gekenn-**zeichnet, dass die kondensierte Probe an einer flüssigkeitsundurchlässigen, aber gasdurchlässigen Membran vorbeigeführt wird, die gasförmigen Oxidationsprodukte in ihrer Gasphase aus der Probe durch die Membran abgesaugt und durch eine Messzelle geleitet werden.

9.  Verfahren nach Anspruch 8, **dadurch gekennzeichnet, dass** die Probe im Bereich der Membran auf einer konstanten Temperatur und einem vorgegebenen Druck gehalten wird.

10. Verfahren nach einem der Ansprüche 7 bis 9, **dadurch gekennzeichnet, dass** ein Anteil der zu untersuchenden gasförmigen Oxidationsprodukte aus einem Bereich geringen Unterdrucks über einen Druckwandler mit einer Blende zu einer Detektionseinheit gelangt.

11. Verfahren nach einem der Ansprüche 7 bis 10, **dadurch gekennzeichnet, dass** die gasförmigen Oxidationsprodukte mittels eines Massenspektrometers gemessen werden.

12. Vorrichtung zur kontinuierlichen Bestimmung gasförmiger Oxidationsprodukte aus Prozeß-, Trink-und/-oder Abwasser, insbesondere zur Bestimmung des TOC-Wertes, mit einer Oxidations- und einer nachgeordneten Meßeinheit, wobei die Oxidationseinheit eine Hochdruckpumpe (3) zum kontinuierlichen Zuführen der Probelösung (2) vorgeordnet ist, **gekennzeichnet durch** einen endseitig geöffneten, beheizbaren Mikroreaktor (4, 4') als Oxidationseinheit in Form einer Heizkapillare zur direkten und kontinuierlichen oxidativen Umsetzung des Kohlenstoffs der zu untersuchenden Probe unter überkritischen Bedingungen.

13. Vorrichtung nach Anspruch 12, **dadurch gekennzeichnet, dass** die Kapillare (4) selbst als Heizwiderstand ausgebildet ist.

14. Vorrichtung nach Anspruch 12, **dadurch gekennzeichnet, dass** die Kapillare (4') mit Heizkörpermaterial einer Widerstandsheizung in wärmeleitender Verbindung steht.

15. Vorrichtung nach einem der Ansprüche 12 bis 14, **gekennzeichnet durch** einen dem Mikroreaktor (4) vorgeordneten elektrochemischen Reaktor (10) zur Sauerstofferzeugung.

16. Vorrichtung nach einem der Ansprüche 12 bis 14, **gekennzeichnet durch** im Inneren des Mikroreaktors angeordnete Elektroden zur elektrochemischen Sauerstofferzeugung.

17. Vorrichtung nach einem der Ansprüche 12 bis 14, **gekennzeichnet durch** eine Gaszuführung zur Einspeisung von Sauerstoff.

18. Vorrichtung nach einem der Ansprüche 12 bis 17, **gekennzeichnet durch** ein dem Mikroreaktor (4) nachgeschaltetes Druckhalteventil (17), wie ein Regulierventil.

19. Vorrichtung nach einem der Ansprüche 12 bis 18, **dadurch gekennzeichnet, dass** die Innenrohrwände des Mikroreaktors (4, 4') temperatur- und korrosionsbeständig beschichtet sind.

20. Vorrichtung nach Anspruch 19, **dadurch gekennzeichnet, dass** das Beschichtungsmaterial eine katalytisch wirkende Metalllegierung ist.

21. Vorrichtung nach Anspruch 19, **dadurch gekennzeichnet, dass** das Beschichtungsmaterial Nickel ist.

22. Vorrichtung nach Anspruch 19, **dadurch gekennzeichnet, dass** das Beschichtungsmaterial Tantal ist.

23. Vorrichtung nach Anspruch 19 oder 20, **dadurch gekennzeichnet, dass** das Beschichtungsmaterial eine Nickellegierung ist.

24. Vorrichtung nach einem der Ansprüche 12 bis 23, **dadurch gekennzeichnet, dass** die Rohraußenwand des Mikroreaktors (4, 4') aus temperaturbeständigem Stahl besteht.

25. Vorrichtung nach einem der Ansprüche 12 bis 23, **dadurch gekennzeichnet, dass** die Rohraußenwand des Mikroreaktors (4, 4') aus einer Keramik ist.

26. Vorrichtung nach einem der Ansprüche 12 bis 25, **gekennzeichnet durch** eine dem Mikroreaktor (4, 4') nachgeordnete Kondensationseinheit (7).

27. Vorrichtung nach einem der Ansprüche 12 bis 26, **gekennzeichnet durch** eine Pumpeinrichtung (11) zum Pumpen der kondensierten Probe (8) **durch** eine Durchflusskammer oder -zelle (12).

28. Vorrichtung nach Anspruch 27, **dadurch gekennzeichnet, dass** der Durchflusszelle (12) ein Druckhalteventil (15) nachgeordnet ist.

29. Vorrichtung nach einem der Ansprüche 21 bis 28, **gekennzeichnet durch** eine Einspeisungsstelle (13') zur Säureeinspeisung zwischen der Kondensationseinheit (7) und der Durchflusszelle (12).

30. Vorrichtung nach einem der Ansprüche 27 bis 29, **dadurch gekennzeichnet, dass** die Durchflusszelle (12) durch eine flüssigkeitsundurchlässige, aber gasdurchlässige Membran von einer Messzelle getrennt ist.

31. Vorrichtung nach einem der Ansprüche 27 bis 30, **dadurch gekennzeichnet, dass** an der Durchflusszelle (12) eine geregelte Heizanordnung angeordnet ist.

32. Vorrichtung nach einem der Ansprüche 30 oder 31, **dadurch gekennzeichnet, dass** der Membran direkt eine einen mäßigen Unterdruck herstellende Pumpe nachgeordnet ist.

33. Vorrichtung nach einem der Ansprüche 27 bis 32, **gekennzeichnet durch** einen Druckwandler mit einstellbarer Blende.

34. Vorrichtung nach einem der Ansprüche 30 bis 33, **dadurch gekennzeichnet, dass** die Messzelle eine Elektronenstoßionenquelle eines Quadrupolmassenspektrometers (14) aufweist.

**Claims**

1. Method for the continuous determination of gaseous oxidation products from process, drinking and/or waste water, in particular for determination of the TOC level, wherein at least organically bonded carbon contained in a sample solution to be examined is oxidatively converted and the gaseous oxidation products are fed to a measurement unit, in which at least the detection of the organically bonded carbon occurs, the sample solution is continuously heated to a predetermined temperature, the organic compounds in the sample solution are continuously oxidatively converted at specifically elevated temperature and specifically elevated pressure, **characterised in that** the sample solution is continuously pumped into a microreactor and heated there, that the elevated pressure is built up by the evaporation pressure of the sample solution such that the carbon of the sample to be examined is oxidatively converted directly and continuously under supercritical conditions.

2. Method according to Claim 1, **characterised in that** the elevated pressure is built up with the aid of a pressure holding valve.

3. Method according to Claim 1 or 2, **characterised in that** oxygen is fed to the sample solution.

4. Method according to Claim 3, **characterised in that** the oxygen is generated electrochemically.

**5.** Method according to one of Claims 1 to 4, **characterised in that** the gaseous sample generated in the microreactor is condensed.

**6.** Method according to Claim 5, **characterised in that** acid is apportioned to the condensed sample.

**7.** Method according to one of Claims 5 or 6, **characterised in that** the condensed sample is directed through a flow cell at a constant flow rate and under constant pressure.

**8.** Method according to Claim 7, **characterised in that** the condensed sample is directed past a membrane, which is liquid-impermeable but gas-permeable, the gaseous oxidation products are extracted from the sample in their gas phase through the membrane and directed through a measurement cell.

**9.** Method according to Claim 8, **characterised in that** the sample is held at a constant temperature and a predetermined pressure in the region of the membrane.

**10.** Method according to one of Claims 7 to 9, **characterised in that** a proportion of the gaseous oxidation products to be examined passes out of a region of low underpressure to a detection unit via a pressure converter with a diaphragm.

**11.** Method according to one of Claims 7 to 10, **characterised in that** the gaseous oxidation products are measured by means of a mass spectrometer.

**12.** Apparatus for the continuous determination of gaseous oxidation products from process, drinking and/or waste water, in particular for determination of the TOC level, with an oxidation unit and a measurement unit connected downstream, wherein the oxidation unit has a high-pressure pump (3) located upstream of it for continuous feed of the sample solution (2), **characterised by** a microreactor (4, 4'), which may be heated and is open at the ends, as oxidation unit in the form of a heating capillary tube for direct and continuous oxidative conversion of the carbon of the sample to be examined under supercritical conditions.

**13.** Apparatus according to Claim 12, **characterised in that** the capillary tube (4) is itself configured as a heating resistor.

**14.** Apparatus according to Claim 12, **characterised in that** the capillary tube (4') is connected in a thermally conductive manner to heating body material of a resistor heating means.

**15.** Apparatus according to one of Claims 12 to 14, **characterised by** an electrochemical reactor (10) disposed upstream of the microreactor (4) for generating oxygen.

**16.** Apparatus according to one of Claims 12 to 14, **characterised by** electrodes disposed inside the microreactor for generating oxygen electrochemically.

**17.** Apparatus according to one of Claims 12 to 14, **characterised by** a gas feed means for supplying oxygen.

**18.** Apparatus according to one of Claims 12 to 17, **characterised by** a pressure holding valve (17), such as a regulating valve, connected downstream of the microreactor (4).

**19.** Apparatus according to one of Claims 12 to 18, **characterised in that** the inside tube walls of the microreactor (4, 4') are provided with a temperature- and corrosion-resistant coating.

**20.** Apparatus according to Claim 19, **characterised in that** the coating material is a catalytically active metal alloy.

**21.** Apparatus according to Claim 19, **characterised in that** the coating material is nickel.

**22.** Apparatus according to Claim 19, **characterised in that** the coating material is tantalum.

**23.** Apparatus according to Claim 19 or 20, **characterised in that** the coating material is a nickel alloy.

**24.** Apparatus according to one of Claims 12 to 23, **characterised in that** the outer tube wall of the microreactor (4, 4') is made of temperature-resistant steel.

**25.** Apparatus according to one of Claims 12 to 23, **characterised in that** the outer tube wall of the microreactor (4, 4') is made of a ceramic.

**26.** Apparatus according to one of Claims 12 to 25, **characterised by** a condensation unit (7) disposed downstream of the microreactor (4, 4').

**27.** Apparatus according to one of Claims 12 to 26, **characterised by** a pumping means (11) for pumping the condensed sample (8) through a flow chamber or cell (12).

**28.** Apparatus according to Claim 27, **characterised in that** a pressure holding valve (15) is disposed downstream of the flow cell (12).

**29.** Apparatus according to one of Claims 21 to 28, **characterised by** a feeding area (13') for supplying acid between the condensation unit (7) and the flow cell (12).

**30.** Apparatus according to one of Claims 27 to 29, **characterised in that** the flow cell (12) is separated from a measurement cell by a membrane, which is liquid-impermeable but gas-permeable.

**31.** Apparatus according to one of Claims 27 to 30, **characterised in that** a regulated heating arrangement is disposed on the flow cell (12).

**32.** Apparatus according to one of Claims 30 or 31, **characterised in that** a pump generating a moderate underpressure is disposed directly downstream of the membrane.

**33.** Apparatus according to one of Claims 27 to 32, **characterised by** a pressure converter with adjustable diaphragm.

**34.** Apparatus according to one of Claims 30 to 33, **characterised in that** the measurement cell has an electron impact ion source of a quadrupole mass spectrometer (14).

**Revendications**

**1.** Procédé pour la détermination continue de produits d'oxydation gazeux à partir d'eaux de processus, potable et/ou usée, en particulier pour la détermination de l'indice CTO, dans lequel du carbone lié organiquement, contenu au moins dans une solution échantillon à étudier est soumis à une conversion oxydative et les produits d'oxydation gazeux sont envoyés à une unité de mesure dans laquelle s'effectue la détection du carbone lié organiquement, la solution échantillon est chauffée continuellement à une température prédéterminée, les composés organiques contenus dans la solution échantillon subissent une conversion oxydative sous une température élevée définie et sous une pression élevée définie continuellement, **caractérisé en ce que** la solution échantillon est refoulée continuellement dans un microréacteur et elle y est chauffée, la pression élevée est établie par la pression de vaporisation de la solution échantillon de telle manière que le carbone de l'échantillon à étudier soit converti par conversion oxydative directement et de façon continue, dans des conditions supercritiques.

**2.** Procédé selon la revendication 1, **caractérisé en ce que** la pression élevée est établie au moyen d'une soupape de retenue de pression.

**3.** Procédé selon la revendication 1 ou 2, **caractérisé en ce que** de l'oxygène est envoyé à la solution échantillon.

**4.** Procédé selon la revendication 3, **caractérisé en ce que** l'oxygène est produit par voie électrochimique.

**5.** Procédé selon la revendication 1 à 4, **caractérisé en ce que** l'échantillon gazeux produit dans le microréacteur est condensé.

**6.** Procédé selon la revendication 5, **caractérisé en ce que** de l'oxygène est ajouté en quantité dosée à l'échantillon condensée.

**7.** Procédé selon une des revendications 5 ou 6, **caractérisé en ce que** l'échantillon condensé est envoyé à une vitesse d'écoulement constante et sous une pression constante à travers une cellule à passage traversant.

**8.** Procédé selon la revendication 7, **caractérisé en ce que** on fait passer l'échantillon condensé sur une membrane imperméable ou liquide mais perméable au gaz, les produits d'oxydation gazeux sont aspirés, dans leur face gazeuse, de l'échantillon à travers une membrane et conduit à travers une cellule de mesure.

**9.** Procédé selon la revendication 8, **caractérisé en ce que** l'échantillon est maintenu dans la région de la membrane à une température constante et à une pression prédéterminée.

**10.** Procédé selon une des revendications 7 à 9, **caractérisé en ce que** une fraction des produits d'oxydation gazeux à étudier parvient, à partir d'une zone de faible dépression, et à travers un convertisseur de pression muni d'un diaphragme, à une unité de dépression.

**11.** Procédé selon une des revendications 7 à 10, **caractérisé en ce que** les produits d'oxydation gazeux sont mesurés à l'aide d'un spectromètre de masse.

**12.** Dispositif pour la détermination continu de produit d'oxydation gazeux ici d'eaux de processus potable et/ou usées, en particulier pour la détermination de l'indice CTO, comprenant une unité d'oxydation et une unité de mesure placée en aval, une pompe à haute pression (3) destinée à amener la solution échantillon (2) en continu et montée en amont de l'unité d'oxydation,
**caractérisé par**
un microréacteur (4, 4') ouvert à une extrémité, pouvant être chauffé, servant d'unité d'oxydation, présenté sous la forme d'un capillaire chauffant destiné à la conversion oxydative direct et continu du carbone de l'échantillon à examiner dans des conditions super critique.

13. Dispositif selon la revendication 12,
**caractérisé en ce que**
le capillaire (4) constitue lui-même une résistance chauffante.

14. Dispositif selon la revendication 12,
**caractérisé en ce que**
le capillaire (4') est en liaison conductrice de la chaleur avec une matire de corps de chauffe appartenant à un chauffage à résistance.

15. Dispositif selon une des revendications 12 à 14,
**caractérisé par**
un réacteur (10) électrochimique, destiné à la production d'oxygène, placé en amont du microréacteur (4).

16. Dispositif selon une des revendications 12 à 14,
**caractérisé par**
des électrodes pour la production d'oxygène électrochimique disposées à l'intérieur du microréacteur.

17. Dispositif selon une des revendications 12 à 14,
**caractérisé par**
une amenée de gaz pour l'injection d'oxygène.

18. Dispositif selon une des revendications 12 à 17,
**caractérisé par**
une soupape de retenue de pression (17) comme une soupape de régulation placée en aval du microréacteur (4).

19. Dispositif selon une des revendications 12 à 18,
**caractérisé en ce que**
les parois intérieures de tube du microréacteur (4, 4') sont revêtues de manière à être résistantes à la température et à la corrosion.

20. Dispositif selon la revendication 19,
**caractérisé en ce que**
la matière de revêtement est un alliage métallique à action catalytique.

21. Dispositif selon la revendication 19,
**caractérisé en ce que**
la matière de revêtement est le nickel.

22. Dispositif selon la revendication 19,
**caractérisé en ce que**
la matière de revêtement est le tantale.

23. Dispositif selon la revendication 19 ou 20,
**caractérisé en ce que**
la matière de revêtement est un alliage de nickel.

24. Dispositif selon une des revendications 12 à 23,
**caractérisé en ce que**
la paroi extérieure du tube du microréacteur (4, 4') est composée d'acier résistant à la température.

25. Dispositif selon une des revendications 12 à 23,
**caractérisé en ce que**
la paroi extérieure du tube du microréacteur (4, 4') est fait d'une céramique.

26. Dispositif selon une des revendications 12 à 25,
**caractérisé par**
une unité de condension (17) placée en aval du microréacteur (4, 4').

27. Dispositif selon une des revendications 12 à 26,
**caractérisé par**
un dispositif de pompe (11) destiné à refouler l'échantillon (8) à travers une chambre ou cellule à passage traversant (12).

28. Dispositif selon une des revendications 27,
**caractérisé par**
une soupape de retenue de la pression (15) placée en aval de la cellule à passage traversant (12).

29. Dispositif selon une des revendications 21 à 28,
**caractérisé par**
une zone d'injection (13') pour l'injection d'oxygène entre l'unité de condensation (7) et la cellule à passage traversant (12).

30. Dispositif selon une des revendications 27 à 29,
**caractérisé en ce que**
la cellule à passage traversant (12) est séparée d'une cellule de mesure par une membrane imperméable au liquide mais perméable au gaz.

31. Dispositif selon une des revendications 27 à 30,
**caractérisé en ce que**
une dispositif de chauffage réglé est agencé sur la cellule à passage traversant (12).

32. Dispositif selon une des revendications 30 à 31,
**caractérisé en ce que**
une pompe produisant une dépression modérée est placée directement en aval de la membrane.

33. Dispositif selon une des revendications 27 à 32,
**caractérisé par**

un convertisseur de pression à diaphragme réglable.

34. Dispositif selon une des revendications 30 à 33, **caractérisé en ce que** la cellule de mesure présente une source d'ions à choc d'électron appartenant à un spectromètre de masse quadripolaire (14).

Figur 1

Figur. 2

Figur 3